# EUROPÄISCHE PATENTSCHRIFT

(11) **EP 1 126 881 B1**
(45) Veröffentlichungstag und Bekanntmachung des Hinweises auf die Patenterteilung: **16.06.2004**
(21) Anmeldenummer: 99953421.7
(22) Anmeldetag: 04.11.1999
(51) Int. Cl.: A61K 47/34

(54) **MUCOADHÄSIVE POLYMERE, DEREN VERWENDUNG SOWIE DEREN HERSTELLUNGSVERFAHREN**
MUCO-ADHESIVE POLYMERS, USE THEREOF AND METHOD FOR PRODUCING THE SAME
POLYMERES MUCO-ADHESIFS, LEUR UTILISATION ET LEUR PROCEDE DE PREPARATION

(30) Priorität: 04.11.1998 AT 182898
(43) Veröffentlichungstag der Anmeldung: 29.08.2001
(73) Patentinhaber: Bernkop-Schnürch, Andreas, 1050 Wien (AT)
(72) Erfinder: Bernkop-Schnürch, Andreas, 1050 Wien (AT)
(74) Vertreter: Alge, Daniel, Mag. Dr. rer.nat.
(86) Internationale Anmeldenummer: PCT/AT1999/000265
(87) Internationale Veröffentlichungsnummer: WO 2000/025823

(56) Entgegenhaltungen:
- EP-A- 0 556 110
- EP-A- 0 635 276
- US-A- 5 665 383
- LUESSEN H L ET AL: "MUCOADHESIVE POLYMERS IN PERORAL PEPTIDE DRUG DELIVERY. IV. POLYCARBOPHIL AND CHITOSAN ARE POTENT ENHANCERS OF PEPTIDE TRANSPORT ACROSS INTESTINAL MUCOSAE IN VITRO.RS OF PEPTIDE" JOURNAL OF CONTROLLED RELEASE,NL,ELSEVIER SCIENCE PUBLISHERS B.V. AMSTERDAM, Bd. 45, Nr. 1, 3. März 1997 (1997-03-03), Seiten 15-23, XP000640521 ISSN: 0168-3659
- TOBYN M J ET AL: "FACTORS AFFECTING IN VITRO GASTRIC MUCOADHESION II. PHYSICAL PROPERTIES OF POLYMERS" EUROPEAN JOURNAL OF PHARMACEUTICS AND BIOPHARMACEUTICS,NL,ELSEVIER SCIENCE PUBLISHERS B.V., AMSTERDAM, Bd. 42, Nr. 1, 1. Januar 1996 (1996-01-01), Seiten 56-61, XP000555023 ISSN: 0939-6411 in der Anmeldung erwähnt

## Beschreibung

Die Erfindung betrifft mucoadhäsive Polymere, Arzneimittel umfassend derartige Polymere sowie Verwendungen von mucoadhäsiven Polymeren.

Seit der Einführung des Konzepts der Bioadhäsion in der pharmazeutischen Literatur wurden viele Versuche im universitären als auch im industriellen Bereich unternommen, um die bioadhäsiven Eigenschaften von verschiedenen Polymeren zu verbessern. Diese Versuche umfassten die Neutralisierung von ionogenen Polymeren (Tobyn et al., Eur.J.Pharm.Biopharm. 42 (1996) 56-61), die Fällung von Polymeren in organischen Lösungsmitteln und deren Lufttrocknung anstelle von Lyophilisierung (Bernkop-Schnürch et al., Int.J.Pharm. 165 (1998) 217-225), Entwicklung von Polymer-Lektin Konjugaten (Naisbett et al., Int.J.Pharm. 107 (1994) 223-230) sowie Konjugate aus Polymeren und bakteriellem Adhesin (Bernkop-Schnürch et al., J.Pharm.Sci. 3 (1995) 293-299).

Diese beschriebenen Systeme basieren alle auf der Bildung von nicht-kovalenten Bindungen, wie z.B. Wasserstoffbrücken oder ionische Wechselwirkungen, mit welchen nur eine schwache Bindung ermöglicht wird, die in vielen Fällen für eine zufriedenstellende Lokalisierung des Wirkstoff-Abgabe-Systems an einem bestimmten Zielort nicht ausreicht.

Jene Mucus-Schicht, die GI-Epithelien überzieht, besteht hauptsächlich aus Mucus-Glycoproteinen, die einen zentralen Bereich mit vielen O-verknüpften Oligosaccharidketten und zwei flankierende cysteinreiche Subdomänen auf jeder Seite aufweisen. Diese cysteinreichen Subdomänen enthalten über 10% Cys in deren Primärstruktur, welche bei der Verknüpfung von Mucinmonomeren zu Oligomeren über Disulfidbrücken eingebunden sind. Damit wird ein dreidimensionales Netzwerk der Mucusgelschicht aufgebaut.

In der EP 0 556 110 A werden starre Vektorsysteme aus porösen Polymerpartikeln für therapeutische und kosmetische Wirkstoffe beschrieben, die auf Basis von Trimethoxysilanen aufgebaut sind und S-S-Bindungen enthalten. In der EP 0 635 276 werden biologische Klebesysteme beschrieben. Keines dieser Dokumente betrifft allerdings Substanzen mit mucoadhäsiven Eigenschaften.

Die Aufgabe der vorliegenden Erfindung besteht im Zur-Verfügung-Stellen von verbesserten mucoadhäsiven Polymeren, mit welchen eine zielgerichtete Einbringung von Wirkstoff in Mucus-Schichten ermöglicht wird, wobei eine stabile Präsenz am Zielort ermöglicht werden soll. Mit der Erfindung soll ein wirksames und effizientes Wirkstoff-Abgabe-System ermöglicht werden, mit welchem eine verbesserte und damit auch verlängerte Anhaftung von Arzneimittel an Schleimhäuten erzielt werden kann.

Fortsetzung auf Seite 2 der bisherigen Beschreibung !

Diese Aufgabe wird erfindungsgemäß durch ein mucoadhäsives Polymer gelöst, welches dadurch gekennzeichnet ist, dass es aus nicht mehr als 10 verschiedenen Monomeren aufgebaut ist und mindestens eine nicht endständige Thiol-Gruppe aufweist. Mit der gezielten Einführung von Thiol-Gruppen in Polymeren mit bekannter mucoadhäsiver Eigenschaft oder mit der Schaffung völlig neuer Thiol-hältiger Polymere wird die spezifische Struktur von Mucus-Schichten in spezifischer Weise ausgenutzt: Es war bekannt, dass die mucolytische Aktivität von Thiolen, wie beispielsweise N-Acetylcystein, auf Disulfidaustauschreaktionen zwischen Glycoproteinen im Mucus und dem mucolytischen Wirkstoff basiert. Aufgrund derartiger Austauschreaktionen werden sowohl intra- als auch intermolekulare Disulfidbrücken in der Glycoproteinstruktur des Mucus gespalten, wodurch die Mucus-Schicht aufgelöst wird. Basierend auf dieser Beobachtung, wonach ein mucolytisches Mittel kovalent an Glycoproteine im Mucus gebunden wird, wurde erfindungsgemäß die Hypothese aufgestellt, dass andere Thiol-hältige Verbindungen, insbesondere Polymere mit Thiol-Gruppen, ebenfalls kovalent an eine Mucus-Schicht gebunden werden könnten. Überraschenderweise hat sich herausgestellt, dass diese Hypothese nicht nur voll zutrifft sondern auch so spezifisch wirkt, dass damit ein effizientes Wirkstoff-Abgabe-System zur Verfügung gestellt werden kann. Insbesondere zeigt es sich, dass die erfindungsgemäßen Polymere im Gegensatz zu den mucolytischen Thiolen keine maßgebliche mucolytische Aktivität aufweisen.

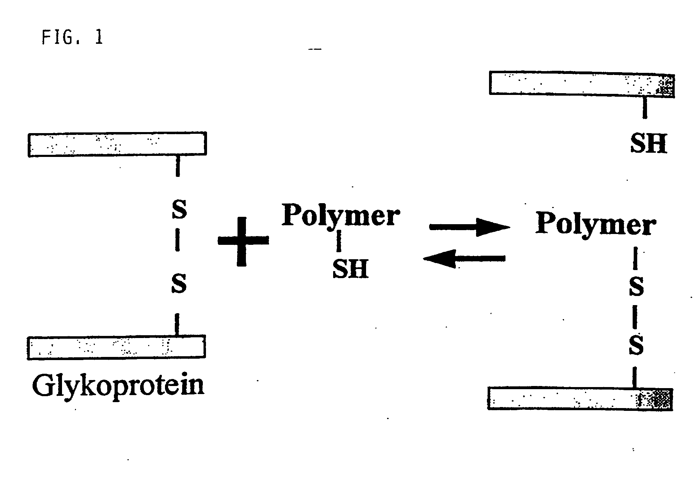

Es zeigte sich, dass die erfindungsgemäßen Polymere in der Lage sind mit den cysteinreichen Subdomänen der Mucusglycoproteine reversible kovalente Bindungen einzugehen (vgl. Fig. 1), mit welchen eine stabile Lokalisierung der Polymere an bestimmten Schleimhäuten im Mucus ermöglicht wird.

Bevorzugterweise enthalten die erfindungsgemäßen Polymere mindestens 0,05 µmol, insbesondere mindestens 0,1 µmol kovalent gebundene Thiol-Gruppen pro g Polymer. Üblicherweise enthalten die erfindungsgemäßen Polymere 1-500 µmol Thiol-Gruppen pro g Polymer, insbesondere 10-100 µmol. Damit wird nicht nur eine effiziente Bindung an die Mucusglycoproteine ermöglicht sondern es werden die mucoadhäsiven Eigenschaften auch noch durch vorteilhafte Hydrationseffekte und interne Kohäsion verstärkt.

Vorzugsweise werden die erfindungsgemäßen Polymere durch Thiolierung von Polymeren hergestellt für die eine mucoadhäsive Eigenschaft bereits bekannt ist. Dabei wird diese mucoadhäsive Eigenschaft entscheidend verstärkt und verbessert. Bevorzugt wird das erfindungsgemäße Polymer daher ausgewählt aus thioliertem Polycarbophil (einem Copolymer aus Acrylsäure und Divinylglykol), thioliertem Chitosan, thiolierter Natrium-Carboxymethylzellulose, thioliertem Natrium-Alginat, thiolierter Natrium-Hydroxypropylzellulose, thiolierte Hyaluronsäure und thioliertem Pektin. Für die unthiolierten Basispolymere ist die mucoadhäsive Eigenschaft beispielsweise in Smart et al. (J.Pharm.Pharmacol. 36 (1984) 295-299) beschrieben.

Selbstverständlich sind auch die thiolierten Derivate der oben genannten Polymere bevorzugt. Beispiele für derartige Derivate umfassen Derivate, die durch Autocrosslinking, Einführung von funktionellen Gruppen, Anbindung von Komplexbildnern (wie z.B. EDTA), Kopplung von Enzyminhibitoren, etc., erhalten werden, insbesondere bei Polymeren mit negativ geladenen Gruppen, z.B. COO⁻-Gruppen.

Die Thiolierung kann erfindungsgemäß durch alle möglichen chemischen Reaktionen vorgenommen werden, mit welchen Thiol-Gruppen an Polymere, insbesondere an wasserlösliche Polymere, gebunden werden. Aus wirtschaftlichen Gründen bietet sich für die Thiolierung die Verwendung von Cystein-Gruppen an, da diese leicht und preisgünsig zu erhalten sind. Cystein-Gruppen können vorzugsweise über eine Amidbindung an das Polymer gebunden werden.

Andererseits kann das erfindungsgemäße Polymer auch dadurch hergestellt werden, dass im Zuge der Herstellung des Polymers mindestens ein Monomer mit Thiol-Gruppen (co-)polymerisiert wird, das im Polymer freie Thiol-Gruppen aufweist d.h. die Thiol-Gruppen nicht in der Polymerisierungsreaktion direkt umgesetzt wird. Ein derartiges Polymer, das mindestens ein Monomer enthält, das im Polymer freie Thiol-Gruppen aufweist, ist erfindungsgemäß ebenfalls bevorzugt.

Bevorzugte erfindungsgemäße Polymere zeichnen sich auch durch eine Bindungskapazität an Darm-Mucosa aus, die gemessen als Gesamt-Adhäsions-Arbeit (total work of adhesion; TWA) größer als 120 µJ ist, insbesondere größer als 150 µJ (bei pH-Wert 7). Ein zur Messung dieser TWA geeignetes System wird in den Beispielen beschrieben.

Bevorzugterweise werden erfindungsgemäß Polymere eingesetzt, die eine im Vergleich zur TWA des nicht-thiolierten Polymers, erhöhte TWA aufweisen. Vorzugsweise beträgt die Erhöhung der TWA 50% oder mehr, insbesondere 100% oder mehr, gemessen am pH-Optimum der TWA des thiolierten Polymers.

Gemäß einem weiteren Aspekt betrifft die vorliegende Erfindung ein Arzneimittel, welches ein erfindungsgemäßes Polymer und zumindest einen Wirkstoff, der über Schleimhäute aufgenommen wird, umfasst. Da mit den erfindungsgemäßen Polymeren gezielt Wirkstoffe an Mucus-Schichten appliziert werden können, ist das erfindungsgemäße Arzneimittel allen bisher bekannten Wirkstoff-Abgabe-Systemen an Mucus-Schichten überlegen, sowohl was seine Spezifität als auch was seine generelle Einsetzbarkeit anbelangt.

Bevorzugterweise wird der Wirkstoff nicht-kovalent an das Polymer gebunden, wodurch die Verabreichung des Wirkstoffes am Zielort durch Diffusion ermöglicht wird. Die Art und Weise wie der Wirkstoff und das Polymer miteinander gemischt oder verknüpft werden ist nicht kritisch, Colyophilisierung ist dabei unter anderem genauso anwendbar wie Lufttrocknen, Gelieren, etc. Auch die Art der Endkonfektionierung des Arzneimittels ist nicht kritisch, bevorzugterweise wird es aber als Tablette, Zäpfchen, Pellet, Augen-, Nasen-, Ohrentropfen oder -gele, als Darreichungsform zur Inhalation oder in Form von Mikro(Nano-)partikel zur Verfügung gestellt.

Als Wirkstoffe kommen bevorzugterweise Wirkstoffe in Betracht, von denen bekannt ist, dass sie eine Wirkung über die Mucus-Schicht zeigen, insbesondere jene Wirkstoffe, die eine vergleichsweise kurze Eliminationshalbwertszeit z.B. unter 3 Stunden im Blut aufweisen. Durch eine verbesserte bzw. verlängerte Anhaftung von Wirkstoffabgabesystemen basierend auf erfindungsgemäßen thiolierten Polymeren, die zudem eine kontrollierte Wirkstofffreigabe über mehrere Stunden ermöglichen, kann die Einnahmefrequenz für solche Wirkstoffe drastisch reduziert werden.

Gemäß einer bevorzugten Ausführungsform enthält das erfindungsgemäße Arzneimittel Wirkstoffe, die von Thiol-Gruppen verstärkt werden, vorzugsweise Thiol-abhängige Enzyme, insbesondere Papain und Subtilisin.

Gemäß einem weiteren Aspekt betrifft die vorliegende Erfindung das erfindungsgemäße Polymer als Arzneimittel bzw. die Verwendung eines erfindungsgemäßen Polymers zur Herstellung eines Arzneimittels, insbesondere eines mucoadhäsiven Arzneimittels. Bevorzugterweise kann dieses Arzneimittel peroral verabreicht werden.

Die erfindungsgemäße Darreichungsform ermöglicht auch eine verzögerte Abgabe des Wirkstoffes, beispielsweise indem der Wirkstoff im Inneren einer Polymertablette zur Verfügung gestellt wird und die Verzögerung durch die Notwendigkeit des Durchtrittes des Wirkstoffes durch die Polymer-Hüllschicht eintritt. Hierbei spielt insbesondere das aufgrund der Thiol-Gruppen verbesserte Quellverhalten der erfindungsgemäßen Polymere eine wichtige Rolle.

Erfindungsgemäß erfolgt die Verabreichung des Arzneimittels in einer effizienten Dosis an Patienten, wobei die Dosis sich nach der im Stand der Technik für den jeweiligen Wirkstoff beschriebenen Dosierung richten kann. Dabei sind aber zwei Aspekte zu berücksichtigen: einerseits ist die erfindungsgemäße Verabreichungsform um einiges gezielter und effizienter als die bekannte Verabreichung (auf demselben Verabreichungsweg), andererseits kann durch die erfindungsgemäßen Polymere die Permeation von Wirkstoffen durch die Mucosa verstärkt werden.

Demgemäß betrifft eine bevorzugte Ausführungsform der vorliegenden Erfindung die Verwendung des erfindungsgemäßen Polymers zur Herstellung eines Mittels zur Verstärkung der Permeation von Wirkstoffen, insbesondere von hochmolekularen, hydrophilen Wirkstoffen, beispielsweise (Poly-)Peptid-Wirkstoffen, durch Mucosa, vorzugsweise durch die Darm-Mucosa.

Es zeigte sich, dass die erfindungsgemäßen Polymere auch in der Lage sind bestimmte Ionen, insbesondere Zink-Ionen zu binden. Durch die Verabreichung des erfindungsgemäßen Polymers werden dabei am Ort der Adhäsion Zink-Ionen vom Polymer gebunden, wodurch Enzyme, insbesondere Enzyme, die Zink-Ionen abhängig sind, inhibiert werden. Eine Inhibierung von Enzymen kann auch durch direkte Bindung der Enzyme an das erfindungsgemäße Polymer erfolgen. Die vorliegende Erfindung betrifft daher auch die Verwendung des erfindungsgemäßen Polymers zur Herstellung eines Mittels zur Inhibierung von Enzymen, insbesondere von Enzymen, die Zink-Ionen abhängig sind. Beispiele hierfür sind vor allem Zink-abhängige Enzyme im Verdauungstrakt, wie Carboxypeptidase A und B.

Gemäß einem weiteren Aspekt betrifft die vorliegende Erfindung auch die Verwendung der erfindungsgemäßen Polymere an nicht-mucosen Kontaktschichten, worin ebenfalls die verbesserten Haftungseigenschaften an biologischem (proteinhaltigen) Material ausgenützt wird. Dabei kommen insbesondere Anwendungen in der Viscochirurgie (intraokuläre chirurgische Eingriffe, Katarakt-Behandlung), intradermale Anwendungen (kosmetisch aber auch therapeutisch; z.B. Faltenglätttung oder Gewebeaugmentierung) aber auch intraartikuläre, insbesondere synariale, Anwendungen in Betracht.

Wie oben erwähnt ist die Herstellung der erfindungsgemäßen Polymere nicht kritisch, ein bevorzugtes Verfahren zur Herstellung der erfindungsgemäßen Polymere ist dadurch gekennzeichnet, dass Basispolymere, die aus nicht mehr als 10 verschiedenen Monomeren aufgebaut sind, wobei zumindest eines der nicht endständigen Monomere eine im Polymer freie endständige funktionelle Gruppe I aufweist, mit Thiol-hältigen Verbindungen, die zumindest eine weitere funktionelle Gruppe II aufweisen, umgesetzt werden, wobei die funktionellen Gruppen I und II bei dieser Umsetzung, gegebenenfalls unter Verwendung von Kopplungsreagenzien, eine kovalente Bindung miteinander eingehen.

Bevorzugterweise ist bei diesem Verfahren die funktionelle Gruppe I eine Carboxylgruppe und die funktionelle Gruppe II eine Aminogruppe, bevorzugterweise eine primäre Aminogruppe, wobei eine Amidbindung eingegangen wird. Bei der Umsetzung können vorzugsweise Kopplungsreagenzien, insbesondere Carbodiimide verwendet werden.

Gemäß einer bevorzugten Ausführungsform wird als Thiol-hältige Verbindung eine Mercaptoverbindung mit primärer Aminogruppe eingesetzt, vorzugsweise Cystein oder ein Cysteinderivat.

Die Umsetzung erfolgt bevorzugterweise bei einem pH-Wert zwischen 4 und 8, insbesondere bei 5,5 bis 6,5.

Das erfindungsgemäß hergestellte Polymer kann dabei auf einen bestimmten pH-Wert eingestellt werden, vorzugsweise auf einen pH-Wert zwischen 5 und 9, insbesondere von 6,5 bis 8,5.

Gemäß einem weiteren Aspekt betrifft die vorliegende Erfindung auch ein Verfahren zur Verbesserung der Mucoadhäsion von Polymeren, welches sich dadurch auszeichnet, dass seitenständige Thiol-Strukturen in diese Polymere eingebracht werden, wodurch es zur Ausbildung von Disulfidbrücken zwischen dem Polymer und der Mucus-Schicht kommt.

Die Erfindung wird anhand der nachfolgenden Beispiele und der Zeichnungsfiguren näher erläutert:
Es zeigen: Fig. 1 das Prinzip der kovalenten Bindung der erfindungsgemäßen Polymere an die Mucus-Schicht;
Fig. 2 die Disintegration von thiolierten Polymeren im Vergleich mit den unmodifizierten Polymeren;
Fig. 3 die Freisetzungsprofile von Rifampicin aus thiolierter und nicht-thiolierter CMC (Fig. 3A) und thioliertem und nicht-thioliertem PCP (Fig. 3B);
Fig. 4 die Permeationswirkung über Darm-Mucosa;
Fig. 5 die Vorrichtung zur Messung der mucoadhäsiven Eigenschaften;
Fig. 6 die Bindung von L-Cystein an thioliertem PCP.

### Beispiel 1: Herstellung eines erfindungsgemäßen Polymers

10 g Polycarbophil (Noveon AA1; Firma BF Goodrich) werden portionsweise in 100 ml einer 4%igen (m/m) methanolischen NaOH Lösung unter ständigem Rühren suspendiert. Das dabei entstehende Natriumsalz des Polymers wird abfiltriert und mit Methanol solange gewaschen bis das Filtrat einen neutralen pH-Wert aufweist. Im Anschluss daran wird das Polymer bei Raumtemperatur im Exsiccator getrocknet. Ein Gramm von neutralisiertem Polycarbophil wird in 250 ml demineralisiertem Wasser hydratisiert und die Carbonsäuregruppen des Polymers durch 1-Ethyl-3-(3-dimethylaminopropyl)-Carbodiimid-Hydrochlorid, das in einer Endkonzentration von 50 mM zugesetzt wird, bei Raumtemperatur unter Rühren 45 Minuten lang voraktiviert. Um eine Oxidation des in der Folge zugesetzten L-Cysteins zu verhindern, wird der pH-Wert der Lösung mit 5 N HCl auf pH-Wert 4 eingestellt und mit N₂ 15 Minuten lang begast. Nach der Zugabe von 0,5 g L-Cystein wird der pH-Wert der Lösung gegebenenfalls auf pH-Wert 4-5 mit HCl bzw. NaOH nachjustiert und der Reaktionsansatz 3 Stunden bei Raumtemperatur unter N₂-Begasung gerührt. Das entstandende Polycarbophil-Cystein-Konjugat wird gegen eine wässrige 1 mM HCl und 2 uM EDTA Lösung, zwei Mal gegen das gleiche Dialysemedium jedoch zusätzlich 1% NaCl enthaltend und abschließend erschöpfend gegen 0,5 mM HCl bei 10°C unter Lichtausschluss dialysiert. Im Anschluss daran wird der pH-Wert des Konjugates mit 1 N NaOH auf pH-Wert 5 eingestellt. Das isolierte Konjugat wird bei -30°C gefriergetrocknet. Die Aufbewahrung erfolgt bei 4°C.

Es wurden verschiedene Polycarbophil-(PCP)Cystein-Konjugate hergestellt, welche die folgenden Thiol-Gruppenkonzentrationen (in µmol/g Polymer) aufwiesen: PCP-Cyst 1:4: 142,2±38,0 µmol/g Polymer; PCP-Cyst 1:2: 12,4±2,3; PCP-Cyst 2:1: 5,3±2,4; PCP-Cyst 4:1: 3,2±2,0; PCP-Cyst 8:1: 2,9±1,4; PCP-Cyst 16:1: 0,6±0,7; PCP-Cyst 32:1: 0,3±0,5; Kontrolle: (PCP+Cyst ohne Reaktion): 0,00±0,00 (dadurch zeigte sich die Effizienz der Reinigung).

Speziell die PCP-Cyst Konjugate 1:2 und 1:4 zeigten eine signifikant (>100%) höhere Wasseraufnahmekapazität im Vergleich mit dem nicht-modifizierten Polymer.

In Mucinbindungsstudien (Bindung von porcinem Mucin an die Polymere) konnte gezeigt werden, dass Mucine effektiv an die getesteten Polymer-Cystein-Konjugate gebunden wurden (im Gegensatz zu den nicht-modifizierten Polymeren).

Die Bindungsstärken (TWA) der erfindungsgemäßen Polymere an Mucine der intestinalen Mucosa wurden im Wesentlichen gemäß Ch'ng et al. (J.Pharm.Sci. 74 (1985) 399-405) durchgeführt, wie in Bernkop-Schnürch et al.(Pharm.Res. 16 (6) (1999) 876-881) beschrieben.

Das hier beschriebene Polymer(Polycarbophil)-Cystein-Konjugat zeigt bei Adhäsionstests sowie bei ex-vivo Studien an exzidierter Schweinedünndarm-Mucosa in künstlichem Darmsaft, bestehend aus 50 mM Tris-HCl Puffer pH-Wert 6,8, der 0,9% NaCl enthält, ein deutlich höheres Anhaftungsvermögen als auf dieselbe Weise vorbehandeltes Polycarbophil, an das jedoch kein Cystein kovalent gebunden wurde.

Es konnte gezeigt werden, dass mit den erfindungsgemäßen Polymeren die Adhäsionswirkung gegenüber nicht-modifiziertem Polymer (PCP) zumindest um 100% gesteigert werden konnte. So konnte beispielsweise mit dem Polymer-Cystein-Konjugat 16:1 eine TWA von 191±47 µJ und mit dem 2:1 Konjugat von 280±67 µJ erzielt werden, wohingegen das unmodifizierte Polymer eine TWA von 104±21 µJ aufwies. Es zeigte sich, dass die Zunahme der TWA bei pH-Wert 6,8 ein Optimum aufwies, jedoch sogar bei pH-Wert 3 positive Effekte der thiolierten Verbindung gegenüber dem Ausgangspolymer auftreten.

### Beispiel 2: Untersuchungen zur Disintegration der erfindungsgemäßen Polymere

Erfindungsgemäß hergestelltes Carboxymethylzellulose-Cystein-Konjugat (CMC-Cystein-Konjugat) und PCP-Cystein-Konjugat wurden lyophilisiert und in eine Matrix-Tablettenform gebracht. Ebenso wurden Tabletten mit den entsprechenden nicht-modifizierten Polymeren hergestellt. Die Stabilität der Polymertabletten (30 mg) in 5 ml 50 mM Tris-HCl gepufferter physiologischer Kochsalzlösung (TBS), pH-Wert 6,8 bei 37°C wurde mit einem Disintegrationstestapparat gemäß der europäischen Pharmacopöe mit einer Oszillationsfrequenz von 0,5 pro sec. analysiert.

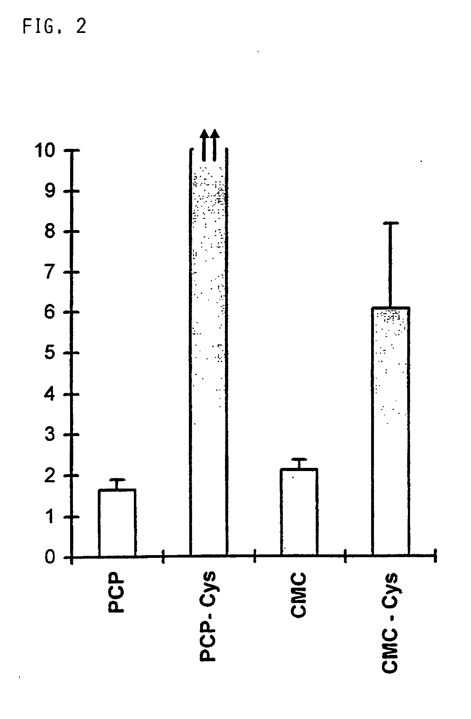

Es zeigte sich, dass die Tabletten aus thiolierten Polymeren eine wesentlich höhere Stabilität als die unmodifizierten Polymere aufwiesen. Matrix-Tabletten, die PCP-Cystein-Konjugate enthalten, waren sogar für mehrere Tage im Test stabil. Die Ergebnisse sind in Fig. 2 dargestellt, wobei auf der y-Achse die Disintegrationszeit in Stunden angegeben ist.

Diese hohe Stabilität der Tabletten der erfindungsgemäßen Polymere kann durch die Ausbildung von Disulfidbrücken in den Polymeren erklärt werden, womit indirekt auch eine verbesserte Anhaftung des Matrixsystems ermöglicht wird, da die Ablösung der Arzneiform von der Schleimhaut durch ein Abreißen der Bindung innerhalb der Arzneiform stark reduziert werden kann. Diese verbesserte Stabilität hat auch wesentliche praktische Bedeutung und bietet verschiedene Vorteile im Vergleich zu bekannten Polymer-Carrier-Systemen, vor allem was die Reduktion von präsystemischen Metabolismen bei Polypeptid-Wirkstoffen im Darm anbelangt.

### Beispiel 3: Freisetzungsuntersuchungen

Erfindungsgemäß hergestellte Konjugate (CMC-Cystein-Konjugat und PCP-Cystein-Konjugat) wurden in demineralisiertem Wasser hydratisiert und in Aceton bzw. 1N NaOH gegeben, wodurch die Viskosität stark erhöht wurde. Nach Waschung mit Aceton bzw. Methanol wurde luftgetrocknet und pulverisiert.

Es wurden Tabletten gefertigt, die aus 1 mg Rifampicin als Modellwirkstoff und 29 mg des CMC-Cystein-Konjugats bzw. des PCP-Cystein-Konjugats sowie den korrespondierenden nicht-modifizierten Polymeren bestehen. Anschließend wurde die in vitro-Freisetzungsrate dieses Wirkstoff-Abgabe-Systems analysiert, indem die Tabletten in 25 ml Behälter, enthaltend 10 ml Freisetzungsmedium (50 mM TBS pH-Wert 6,8), gegeben wurden. Die Behälter wurden verschlossen und auf einem oszillierenden Wasserbad bei 37±0,5°C inkubiert. 600 µl Aliquote wurden in einstündigen Intervallen entnommen und durch gleiche Volumina an Freisetzungsmedium ersetzt. Freigesetztes Rifampicin wurde photometrisch bei 470 nm mittels Eichgerade quantifiziert.

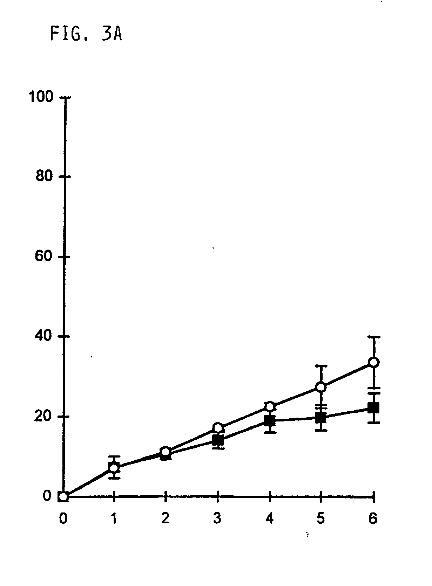

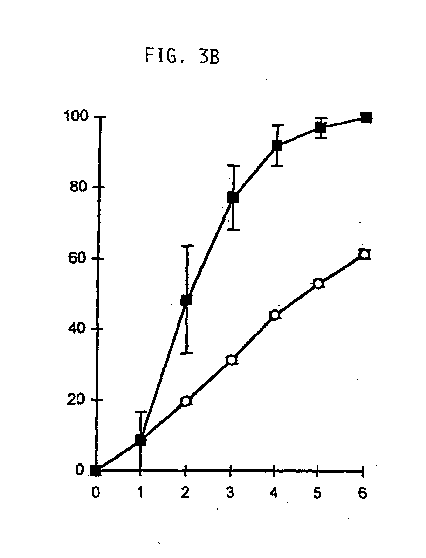

Die Ergebnisse sind in Fig. 3A (für CMC) und Fig. 3B (für PCP) wiedergegeben, wobei auf der x-Achse die Zeit in Stunden und auf der y-Achse die Prozent freigesetztes Rifampicin aufgetragen wurden.

Es zeigte sich, dass mit den erfindungsgemäßen Systemen eine wesentlich effizientere Freisetzung erfolgt, die vor allem in Anbetracht der Disintegrationsergebnisse das große Potential der erfindungsgemäßen Polymere demonstriert. Eine kontrollierte Wirkstofffreisetzung für eine verlängerte Zeitdauer wird dabei in effizienter Weise erreicht.

### Beispiel 4: Wirkung der erfindungsgemäßen Polymere als Permeationsverstärker

2 mg Fluoresceinisothiocyanat (FITC) wurden in 1 ml DMSO aufgelöst und in aliquoten Volumina von 25 µl 40 mg Bacitracin (gelöst in 20 ml 0,1 M Na₂CO₃) zugegeben. Um die Kopplungsreaktion nach 8 Stunden bei 4°C zu stoppen wurde Ammoniumchlorid in einer Endkonzentration von 50 mM zugegeben. Das entstandene FITC-Konjugat wurde durch Gelfiltration über Sephadex G15 isoliert und lyophilisiert.

Permeationsuntersuchungen mit diesem modifizierten Peptid wurden bei 37°C unter Verwendung von Ussing-Kammern mit Dünndarmstücken von Meerschweinchen durchgeführt. Die Donor- und die Akzeptorkammer wurde jeweils mit 1 ml einer Lösung enthaltend 250 mM Natriumchlorid, 2,6 mM Magnesiumsulfat, 10,0 mM Calciumchlorid, 40,0 mM Glucose und 50 mM Natriumhydrogencarbonat (pH-Wert 7,2) gefüllt. Das Bacitracin-FITC-Konjugat wurde der Donorkammer in einer Endkonzentration von 0,1% (m/v) zugegeben. Aliquote Volumina von 200 µl wurden aus der Akzeptorkammer zu bestimmten Zeitpunkten entnommen und durch dasselbe Medium ersetzt. Der Einfluss von PCP und thioliertem PCP (PCP-Cyst), welches erfindungsgemäß hergestellt worden ist, auf das Permeationsverhalten des modifizierten Peptides wurde durch Zugabe von 0,5% (m/v) PCP und 0,5% (m/v) PCP-Cyst untersucht. Die Menge an permeiertem Bacitracin-FITC-Konjugat wurde mit einem Fluorimeter bestimmt. Ebenso wurden die Änderungen in transepitelialen elektrischen Widerstand überwacht.

Es konnte gezeigt werden, dass Bacitracin mit einem Molekulargewicht von 1422 Da die Darm-Mucosa zu einem bestimmten Grad permeieren kann. Dabei konnte eine Degradation durch Verdauungsenzyme aufgrund seiner enzymhemmenden Wirkung ausgeschlossen werden. Die Zugabe von 0,5% PCP führte zu einem 1,2fachen Anstieg des Transports des Modellpeptids durch die Membran, wohingegen die Verwendung der erfindungsgemäß hergestellten Polymere eine signifikant erhöhte Steigerung (etwa das 1,5fache) in der Permeation ermöglichten. Als Gegenversuch konnte gezeigt werden, dass Cystein per se keinen Einfluss auf die Permeation hatte, wodurch der signifikante Effekt der erfindungsgemäßen Polymere belegt wird.

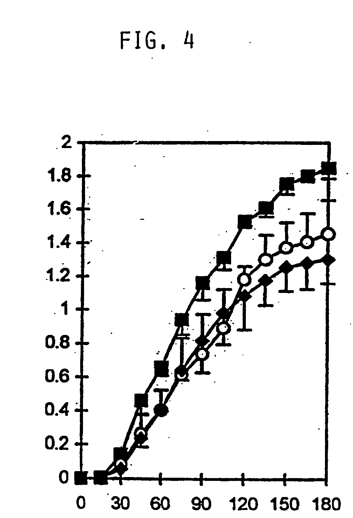

Die Ergebnisse dieses Experiments sind in Fig. 4 dargestellt, wobei auf der x-Achse die Zeit in Minuten und auf der y-Achse die Permeation in Prozent der gesamten Dosis angegeben werden (o: PCP :■PCP-Cyst ◆ Kontrolle).

### Beispiel 5: In vitro-Mucuadhäsionsuntersuchungen

PCP (Molgewicht größer 700 kDa) wurde mit NaOH neutralisiert. Die Carbonsäuregruppen von hydriertem, neutralisiertem PCP und hydriertem CMC (Molgewicht rund 1000 kDa) wurden für 45 Minuten durch Zugabe von 1-Ethyl-3-(3-dimethylaminopropyl)-Carbodiimid-Hydrochlorid (EDAC; Sigma) in einer EndKonzentration von 50 mM aktiviert. L-Cysteinhydrochlorid wurde zugegeben und der pH-Wert der Reaktionsmischung wurde auf 4-5 eingestellt. Das molare Verhältnis von EDAC zu L-Cystein war 50:3,2 und 50:1 für die Kopplungsreaktionen mit PCP bzw. CMC. Der pH-Wert der Kopplungsreaktion mit CMC wurde durch Zugabe von 1 N HCl konstant gehalten. Die Reaktionsmischungen wurden für 3 Stunden bei Raumtemperatur inkubiert. Die erhaltenen Polymer-Cystein-Konjugate wurden durch Dialyse bei 10°C im Dunkeln gegen 1 mN HCl isoliert. Anschließend wurde der pH-Wert dieser Polymere mit 1 N NaOH auf pH 3, pH 5 bzw. pH 7 eingestellt und lyophilisiert. Die erhaltenen thiolierten Polymere wiesen 12,3 µmol (PCP-Konjugat) und 22,3 µmol (CMC-Konjugat) Thiol-Gruppen/g Polymer auf.

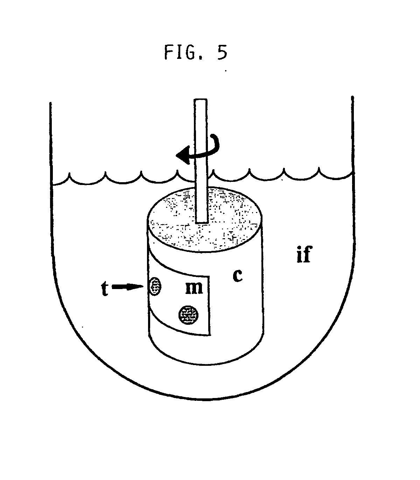

Die Mucuadhäsionsuntersuchungen wurden mit einer Apparatur gemäß US-Pharmacopöe (vgl. Fig. 5) durchgeführt: Dabei wurde eine frisch exzisierte intestinale Mucosa vom Schwein auf einem Stahlzylinder (Durchmesser 4,5 cm, Höhe 5,1 cm, Apparatus 4-Zylinder, USP XXII) aufgespannt. Dieser Zylinder wurde in den Dissolutionsapparat, enthaltend 100 mM TBS pH-Wert 6,8 bei 37°C, eingebracht und mit 250 Umdrehungen/Minute bewegt. Die Polymere wurden zu 30 mg-Tabletten von 5,0 mm Durchmesser gepresst, auf die Mucosa aufgebracht und über einen Zeitraum von 10 Stunden beobachtet. Die Ergebnisse sind in der folgenden Tabelle dargestellt:

| Polymer | pH 3 | pH 5 | pH 7 |
|---|---|---|---|
| PCP Kontrolle | 7,5±1,35 dis. | 4,8±1,35 los. | 4,6±1,39 dis. |
| PCP-Cys | 7,55±1,15 dis. | >10 | 2,25±0,87 los. |
| CMC Kontrolle | 2,0±0,35 los. | 2,5±0,5 dis. | 1,5±0,91 los. |
| CMC-Cys | 3,9±1,02 los. | 3,0±0,35 los. | 1,7±0,57 los. |
| dis..Disintegration los..Loslösung | | | |

Es zeigte sich, dass die erfindungsgemäßen Polymere deutlich verbesserte Eigenschaften gegenüber den nicht-thiolierten Ausgangspolymeren aufwiesen. Es zeigte sich, dass im erfindungsgemäßen System das Zusammenwirken der Eigenschaften:Haftungsfähigkeit an der Mucosa, Bindungsmechanismus der erfindungsgemäßen Polymere an die Mucosa, erhöhte Kohäsivität und Quellverhalten zu einem optimalen Adhäsionsprozess führen, wodurch eine überlegene Arzneimittelzuführung durch optimierte Anhaftung an die zu Mucus-Schichten ermöglicht wird.

### Beispiel 6: Enzyminhibierungswirkungen

Die Inhibierungswirkung von PCP-Cystein-Konjugaten und nicht-modifizierten neutralisierten PCP wurden gegenüber Carboxypeptidase A und Carboxypeptidase B getestet. Hierbei wurde in für diese Enzyme beschriebenen Enzymaktivitätstests untersucht:
0,5 mg der Polymere bzw. von L-Cystein und 0,5 Einheiten Carboxypeptidase A aus Rinderpankreas wurden in 400 µl 25 mM Tris-HCl pH-Wert 6,8, enthaltend 2,9% NaCl, 30 Minuten lang bei Raumtemperatur inkubiert. Nach Zentrifugation wurden 300 µl Überstand in 300 µl 2 mM Hippuryl-L-phenylalanin gegeben, wobei der Anstieg der Absorption bei 254 nm in 1 Minuten-Intervallen gemessen wurde.

Die Polymere (1 mg) und Carboxypeptidase B (0,62 Einheiten) aus Rinderpankreas wurden in einem Gesamtvolumen von 600 µl 30 Minuten bei 37°C inkubiert. Nach Zentrifugation wurden 400 µl Überstand in 400 µl 2 mM Hippuryl-L-arginin gegeben, wobei der Anstieg der Absorption bei 258 nm in 1 Minuten-Intervallen gemessen wurde.

Es zeigte sich, dass der bereits gegebene inhibitorische Effekt von PCP gegenüber Carboxypeptidase A und B signifikant durch die Immobilisierung von Cystein am Polymer erhöht werden konnte. Da die Bindungsaffinität von PCP gegenüber Zink durch die Immobilisierung von Cystein auf dem Polymer um das 1,13fache gesteigert werden konnte (68,7±1,9% Zink werden an PCP gebunden, wohingegen 97,8±0,5 an das PCP-Cystein gebunden werden), und diese Exopeptidasen nicht an die Polymere gebunden werden, ist klar, dass die Verstärkung im inhibierenden Effekt auf die höhere Zinkaffinität der erfindungsgemäßen Polymere zurückzuführen ist.

### Beispiel 7: Cystein-Bindung an das erfindungsgemäße Polymer

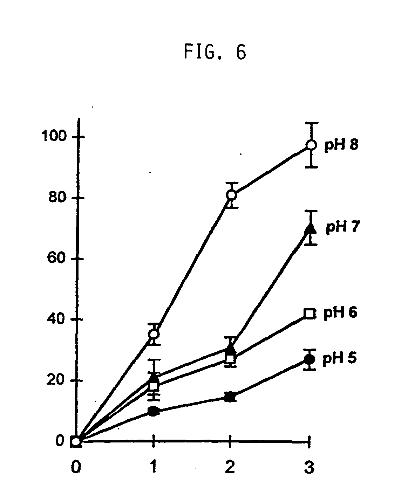

In Cystein-Bindungsstudien wurden 0,5% (m/v) des hergestellten PCP-Cystein-Konjugates und 0,1% (m/v) L-Cystein bei verschiedenen pH-Werten bei 37°C inkubiert. Die Ergebnisse sind in Fig. 6 dargestellt: Auf der x-Achse ist die Zeit in Stunden und auf der y-Achse das gebundene Cystein in % des theoretischen Maximums, das an das Polymer gebunden werden kann, aufgetragen.

Aus diesen Bindungsstudien geht klar hervor, dass die erfindungsgemäßen Polymere an Cystein-Teilstrukturen in biologischen Systemen kovalent anhaften können und somit auch für Anwendungen bei welchen eine verbesserte Haftung an nicht-mucosen Kontaktflächen, wie z.B. bei intradermalen, intraartikulären und intraokulären Anwendungen, geeignet sind.

## Patentansprüche

1. Mucoadhäsives Polymer, **dadurch gekennzeichnet, dass** es
- aus nicht mehr als 10 verschiedenen Monomeren aufgebaut ist und
- mindestens eine nicht endständige Thiol-Gruppe aufweist.

2. Polymer nach Anspruch 1, **dadurch gekennzeichnet, dass** es mindestens 0,05 µmol, insbesondere mindestens 0,1 µmol kovalent gebundene Thiol-Gruppen pro g Polymer enthält.

3. Polymer nach Anspruch 1 oder 2, **dadurch gekennzeichnet, dass** das Polymer ausgewählt ist aus thioliertem Copolymer aus Acrylsäure und Divinylglykol, thioliertem Chitosan, thiolierter Natrium-Carboxymethylzellulose, thioliertem Natrium-Alginat, thiolierter Natrium-Hydroxypropylzellulose, thiolierter Hyaluronsäure und thioliertem Pektin oder Derivaten dieser thiolierten Polymere.

4. Polymer nach einem der Ansprüche 1 bis 3, **dadurch gekennzeichnet, dass** die Thiol-Gruppen Cystein-Gruppen sind, die vorzugsweise über eine Amidbindung an das Polymer gebunden sind.

5. Polymer nach einem der Ansprüche 1 bis 4, **dadurch gekennzeichnet, dass** es mindestens ein Monomer enthält, das im Polymer freie Thiol-Gruppen aufweist.

6. Polymer nach einem der Ansprüche 1 bis 5, **dadurch gekennzeichnet, dass** es eine Gesamt-Adhäsions-Arbeit (TWA) von mehr als 120 µJ, insbesondere von mehr als 150 µJ, an Darm-Mucosa bei pH-Wert 7 aufweist.

7. Polymer nach einem der Ansprüche 1 bis 6, **dadurch gekennzeichnet, dass** es eine gegenüber dem nicht-thiolierten Polymer eine um zumindest 30% erhöhte TWA, gemessen am pH-Optimum der TWA des thiolierten Polymers, aufweist, vorzugsweise eine TWA, die um 50% oder mehr, insbesondere um 100% oder mehr, erhöht ist.

8. Arzneimittel, umfassend ein Polymer nach einem der Ansprüche 1 bis 7 und zumindest einen Wirkstoff, der über Schleimhäute aufgenommen wird.

9. Arzneimittel nach Anspruch 8, **dadurch gekennzeichnet, dass** der Wirkstoff nicht-kovalent an das Polymer gebunden ist.

10. Arzneimittel nach Anspruch 8 oder 9, **dadurch gekennzeichnet, dass** es als Tablette, Zäpfchen, Pellet, Augen-, Nasen-, Ohrentropfen oder -gele, als Darreichungsform zur Inhalation oder in Form von Mikro(Nano)-partikel vorliegt.

11. Arzneimittel nach einem der Ansprüche 8 bis 10, **dadurch gekennzeichnet, dass** es Wirkstoffe umfasst, die von Thiol-Gruppen verstärkt werden, vorzugsweise Thiol-abhängige Enzyme, insbesondere Papain und Subtilisin.

12. Verwendung eines Polymers nach einem der Ansprüche 1 bis 7 zur Herstellung eines Arzneimittels.

13. Verwendung eines Polymers nach einem der Ansprüche 1 bis 7 zur Herstellung eines mucoadhäsiven Arzneimittels.

14. Verwendung eines Polymers nach einem der Ansprüche 1 bis 7 zur Herstellung eines peroral verabreichbaren Arzneimittels.

15. Verwendung nach einem der Ansprüche 12 bis 14, **dadurch gekennzeichnet, dass** ein Arzneimittel hergestellt wird, dessen Wirkstoff verzögert abgegeben wird.

16. Verwendung eines Polymers nach einem der Ansprüche 1 bis 7 zur Herstellung eines Mittels zur Verstärkung der Permeation von Wirkstoffen, insbesondere von (Poly-)Peptid-Wirkstoffen, durch Mucosa, insbesondere durch die Darm-Mucosa.

17. Verwendung eines Polymers nach einem der Ansprüche 1 bis 7 zur Herstellung eines Mittels zur intradermalen, intraokulären oder intraartikulären Anwendung.

18. Verwendung eines Polymers nach einem der Ansprüche 1 bis 6 zur Herstellung eines Mittels zur Inhibierung von Enzymen, insbesondere von Enzymen, die Zink-Ionen abhängig sind.

19. Verfahren zur Herstellung eines Polymers nach einem der Ansprüche 1 bis 6, **dadurch gekennzeichnet, dass** Basispolymere, die aus nicht mehr als 10 verschiedenen Monomeren aufgebaut sind, wobei zumindest eines der nicht endständigen Monomere eine im Polymer freie endständige funktionelle Gruppe I aufweist, mit Thiol-hältigen Verbindungen, die zumindest eine weitere funktionelle Gruppe II aufweist, umgesetzt werden, wobei die funktionellen Gruppen I und II bei dieser Umsetzung, gegebenenfalls unter Verwendung von Kopplungsreagenzien, eine kovalente Bindung miteinander eingehen.

20. Verfahren nach Anspruch 19, **dadurch gekennzeichnet, dass** die funktionelle Gruppe I eine Carboxylgruppe und die funktionelle Gruppe II eine Aminogruppe, vorzugsweise eine primäre Aminogruppe, ist und bei der Umsetzung Kopplungsreagenzien, insbesondere Carbodiimide, verwendet werden, wobei eine Amidbindung eingegangen wird.

21. Verfahren nach Anspruch 19 oder 20, **dadurch gekennzeichnet, dass** als Thiol-hältige Verbindung eine Mercaptoverbindung mit primärer Aminogruppe eingesetzt wird, vorzugsweise Cystein oder ein Cysteinderivat.

22. Verfahren nach einem der Ansprüche 19 bis 21, **dadurch gekennzeichnet, dass** die Umsetzung bei einem pH-Wert zwischen 4 und 8, insbesondere bei 5,5 bis 6,5 durchgeführt wird.

23. Verfahren nach einem der Ansprüche 19 bis 22, **dadurch gekennzeichnet, dass** das hergestellte Polymer auf einen pH-Wert zwischen 5 und 9, insbesondere von 6,5 bis 8,5, eingestellt wird.

24. Verfahren zur Herstellung eines Arzneimittels nach einem der Ansprüche 8 bis 11, **dadurch gekennzeichnet, dass** ein Polymer nach einem der Ansprüche 1 bis 7 mit einem Wirkstoff vereinigt wird.

25. Verfahren nach Anspruch 24, **dadurch gekennzeichnet, dass** bei der Vereinigung der Wirkstoff vom Polymer nicht kovalent gebunden wird.

26. Verfahren nach Anspruch 24 oder 25, **dadurch gekennzeichnet, dass** das Polymer und der Wirkstoff co-lyophilisiert werden.

27. Verfahren zur Verbesserung der Mucoadhäsion von Polymeren, **dadurch gekennzeichnet, dass** seitenständige Thiol-Gruppen in diese Polymere eingebracht werden, wodurch es zur Ausbildung von Disulfidbrücken zwischen dem Polymer und der Mucus-Schicht kommt.

## Claims

1. A mucoadhesive polymer, **characterised in that**
. it is assembled of not more than 10 different monomers and
. comprises at least one non-terminal thiol group.

2. A polymer according to claim 1, **characterised in that** it comprises at least 0.05 µmol, in particular at least 0.1 µmol, of covalently bound thiol groups per gram of polymer.

3. A polymer according to claim 1 or 2, **characterised in that** the polymer is selected from thiolated copolymer of acrylic acid and divinyl glycol, thiolated chitosan, thiolated sodium carboxymethylcellulose, thiolated sodium alginate, thiolated sodium hydroxypropylcellulose, thiolated hyaluronic acid and thiolated pectin or derivatives of these thiolated polymers.

4. A polymer according to any one of claims 1 to 3, **characterised in that** the thiol groups are cysteine groups which preferably are bound to the polymer via an amide bond.

5. A polymer according to any one of claims 1 to 4, **characterised in that** it comprises at least one monomer which comprises free thiol groups in the polymer.

6. A polymer according to any one of claims 1 to 5, **characterised in that** it has a total work of adhesion (TWA) of more than 120 µJ, in particular more than 150 µJ, to intestinal mucosa at pH 7.

7. A polymer according to any one of claims 1 to 6, **characterised in that** compared to the TWA of the non-thiolated polymer, it has an at least 30% increased TWA, measured at the pH optimum of the TWA of the thiolated polymer, preferably, a TWA which is increased by 50% or more, in particular by 100% or more.

8. A drug comprising a polymer according to any one of claims 1 to 7 and at least one active substance which is taken up via the mucosae.

9. A drug according to claim 8, **characterised in that** the active substance is non-covalently bound to the polymer.

10. A drug according to claim 8 or 9, **characterised in that** it is provided as a tablet, suppository, pellet, eye-, nose-, ear-drops or -gels, in a form to be administered by inhaling or in the form of micro(nano) particles.

11. A drug according to any one of claims 8 to 10, **characterised in that** it comprises active substances which are enhanced by thiol groups, preferably thiol-dependent enzymes, in particular papain and subtilisin.

12. The use of a polymer according to any one of claims 1 to 7 for preparing a drug.

13. The use of a polymer according to any one of claims 1 to 7 for preparing a mucoadhesive drug.

14. The use of a polymer according to any one of claims 1 to 7 for preparing a drug for peroral administration.

15. The use of a polymer according to any one of claims 12 to 14, **characterised in that** a drug is prepared whose active substance is released with delay.

16. The use of a polymer according to any one of claims 1 to 7 for preparing an agent for increasing the permeation of active substances, in particular of active (poly)peptide substances, through the mucosa, in particular through the intestinal mucosa.

17. The use of a polymer according to any one of claims 1 to 7 for preparing an agent for intradermal, intraocular or intraar-ticular application.

18. The use of a polymer according to any one of claims 1 to 6 for preparing an agent for inhibiting enzymes, in particular zinc ion-dependent enzymes.

19. A method of preparing a polymer according to any one of claims 1 to 6, **characterised in that** base polymers which are assembled of not more than 10 different monomers, wherein at least one of the non-terminal monomers comprises a terminal functional group I that is free within the polymer, are reacted with thiol-containing compounds comprising at least one further functional group II, the functional groups I and II forming a covalent bond with each other during this reaction, optionally with the use of coupling reagents.

20. A method according to claim 19, **characterised in that** the functional group I is a carboxyl group, and the functional group II is an amino group, preferably a primary amino group, and that coupling reagents, in particular carbodiimides, are used in the reaction, an amide bond being formed.

21. A method according to claim 19 or 20, **characterised in that** a mercapto compound having a primary amino group, preferably cysteine or a cysteine derivative, is used as the thiol-containing compound.

22. A method according to any one of claims 19 to 21, **characterised in that** the reaction is carried out at a pH of between 4 and 8, in particular at pH 5.5 to 6.5.

23. A method according to any one of claims 19 to 22, **characterised in that** the polymer prepared is adjusted to a pH of between 5 and 9, in particular a pH from 6.5 to 8.5.

24. A method for preparing a drug according to any one of claims 8 to 11, **characterised in that** a polymer according to any one of claims 1 to 7 is combined with an active substance.

25. A method according to claim 24, **characterised in that** at combining, the active substance is not covalently bound by the polymer.

26. A method according to claim 24 or 25, **characterised in that** the polymer and the active substance are co-lyophilized.

27. A method of improving the mucoadhesion of polymers, **characterised in that** laterally arranged thiol groups are introduced into these polymers, resulting in the formation of disulfide bonds between the polymer and the mucus layer.

## Revendications

1. Polymère muco-adhésif, **caractérisé en ce que**
- il est constitué d'au plus 10 monomères différents et
- il contient au moins un groupe thiol non terminal.

2. Polymère selon la revendication 1, **caractérisé en ce qu'**il contient au moins 0,05 µmol, en particulier au moins 0,1 µmol de groupes thio liés par covalence par g de polymère.

3. Polymère selon la revendication 1 ou 2, **caractérisé en ce que** le polymère est choisi parmi un copolymère thiolé d'acide acrylique et de divinylglycol, un chitosane thiolé, une carboxyméthylcellulose sodique thiolée, un alginate de sodium thiolé, une hydroxypropylcellulose sodique thiolée, de l'acide hyaluronique thiolé et de la pectine thiolée ou des dérivés de ces polymères thiolés.

4. Polymère selon l'une des revendications 1 à 3, **caractérisé en ce que** les groupes thiol sont des groupes de cystéine qui sont de préférence liés au polymère par l'intermédiaire d'une liaison amide.

5. Polymère selon l'une des revendications 1 à 4, **caractérisé en ce qu'**il contient au moins un monomère présentant dans le polymère des groupes thiol libres.

6. Polymère selon l'une des revendications 1 à 5, **caractérisé en ce qu'**il présente un travail d'adhésion total (TWA) de plus de 120 µJ, en particulier de plus de 150 µJ, sur la muqueuse intestinale à pH 7.

7. Polymère selon l'une des revendications 1 à 6, **caractérisé en ce qu'**il présente par rapport au polymère non thiolé une augmentation d'au moins 30 % du TWA mesuré au pH optimal du TWA du polymère thiolé, de préférence une augmentation du TWA d'au moins 50 %, en particulier d'au moins 100 %.

8. Médicament comprenant un polymère selon l'une des revendications 1 à 7 et au moins une substance active qui est absorbée par l'intermédiaire des muqueuses.

9. Médicament selon la revendication 8, **caractérisé en ce que** la substance active est liée de manière non covalente au polymère.

10. Médicament selon la revendication 8 ou 9, **caractérisé en ce qu'**il se trouve sous forme de comprimés, de suppositoires, de pastilles, de gouttes ou de gels pour les yeux, le nez, les oreilles, sous une forme d'administration à inhaler ou sous forme de micro(nano)particules.

11. Médicament selon l'une des revendications 8 à 10, **caractérisé en ce qu'**il comprend des substances actives qui sont renforcées par des groupes thiol, de préférence des enzymes dépendantes des groupes thiol, en particulier la papaïne et la subtilisine.

12. Utilisation d'un polymère selon l'une des revendications 1 à 7 pour la préparation d'un médicament.

13. Utilisation d'un polymère selon l'une des revendications 1 à 7 pour la préparation d'un médicament muco-adhésif.

14. Utilisation d'un polymère selon l'une des revendications 1 à 7 pour la préparation d'un médicament administrable par voie orale.

15. Utilisation selon l'une des revendications 12 à 14, **caractérisée en ce que** l'on prépare un médicament dont la substance active est libérée de façon retardée.

16. Utilisation d'un polymère selon l'une des revendications 1 à 7 pour la préparation d'un agent destiné à renforcer la pénétration de substances actives, en particulier de substances actives (poly)peptidiques, à travers une muqueuse, en particulier à travers la muqueuse intestinale.

17. Utilisation d'un polymère selon l'une des revendications 1 à 7 pour la préparation d'un agent destiné à l'application intradermique, intraoculaire ou intra-articulaire.

18. Utilisation d'un polymère selon l'une des revendications 1 à 6 pour la préparation d'un agent destiné à inhiber des enzymes, en particulier des enzymes dépendantes des ions zinc.

19. Procédé de préparation d'un polymère selon l'une des revendications 1 à 6, **caractérisé en ce que** l'on fait réagir des polymères de base, constitués d'au plus 10 monomères différents, au moins l'un des monomères non terminaux présentant un groupe fonctionnel I terminal libre dans le polymère, avec des composés thiolés présentant au moins un autre groupe fonctionnel II, grâce à quoi les groupes fonctionnels I et II forment entre eux une liaison covalente dans cette réaction, éventuellement en présence de réactifs de couplage.

20. Procédé selon la revendication 19, **caractérisé en ce que** le groupe fonctionnel I est un groupe carboxy et le groupe fonctionnel II un groupe amino, de préférence un groupe amino primaire, et on utilise dans la réaction des réactifs de couplage, en particulier des carbodiimides, ce qui entraîne la formation d'une liaison amide.

21. Procédé selon la revendication 19 ou 20, **caractérisé en ce que** l'on utilise comme composé thiolé un composé mercapto contenant un groupe amino primaire, de préférence la cystéine ou un dérivé de cystéine.

22. Procédé selon l'une des revendications 19 à 21, **caractérisé en ce que** la réaction s'effectue à un pH compris entre 4 et 8, en particulier de 5,5 à 6,5.

23. Procédé selon l'une des revendications 19 à 22, **caractérisé en ce que** l'on amène le polymère préparé à un pH compris entre 5 et 9, en particulier de 6,5 à 8,5.

24. Procédé de préparation d'un médicament selon l'une des revendications 8 à 11, **caractérisé en ce que** l'on combine un polymère selon l'une des revendications 1 à 7 avec une substance active.

25. Procédé selon la revendication 24, **caractérisé en ce que**, lors de la combinaison, la substance active n'est pas liée de façon covalente par le polymère.

26. Procédé selon la revendication 24 ou 25, **caractérisé en ce que** le polymère et la substance active sont co-lyophilisés.

27. Procédé pour améliorer l'adhésion aux muqueuses de polymères, **caractérisé en ce que** l'on introduit dans ces polymères des groupes thiol latéraux, ce qui entraîne la formation de ponts disulfure entre le polymère et la couche muqueuse.
